# EUROPEAN PATENT APPLICATION

(11) **EP 2 974 650 A1**
(43) Date of publication of application: **20.01.2016**
(21) Application number: 15177308.2
(22) Date of filing: 17.07.2015
(51) Int. Cl.: A61B 5/00, H02J 7/00

(54) **WEARABLE DEVICE AND METHOD FOR MANUFACTURING THE SAME**

(30) Priority: 18.07.2014 CN 201410345261
(71) Applicant: Xiaomi Inc., Beijing 100085 (CN)
(72) Inventor: ZHANG, Yi, 100085 Beijing (CN); XIA, Yongfeng, 100085 Beijing (CN); LI, Ningning, 100085 Beijing (CN); WANG, Tao, 100085 Beijing (CN); ZHANG, Lian, 100085 Beijing (CN)
(74) Representative: Delumeau, François Guy

(57) **Abstract**

This invention relates to a wearable device and its manufacturing method, belonging to a smart wear technical field. The wearable device comprises: a front case (110), a bottom case (130) and electronic components (150); the front case (110) is joined to the bottom case (130) in an up-and-down direction, and the front case (110) and the bottom case (130) together enclose a space forming a waterproof cavity; the electronic components (150) are disposed in the cavity; the electronic components (150) comprise a printed circuit board (PCB) (151) and a battery (152), which are electrically connected, and the PCB (151) is provided with a wireless data transceiving assembly and at least one type of sensor.

## Description

### TECHNICAL FIELD

The invention relates to the technical field of smart wearables, and more particularly, to a wearable device and a method for manufacturing the same.

### BACKGROUND

A wrist band is a decorative article which people are usually fond of wearing, and may serve a decorative function very well. With rapid technical development, however, requirements for functions of a wrist band become higher and higher.

In the prior art, a wrist band is provided with a sensor built in to collect user's health data when the user wears the wrist band, and after that, the user may transmit the health data to other devices through USB (Universal Serial Bus) data line for review and analysis.

During achievement of the invention, it is found that the prior art has at least the following disadvantage: since it is required to provide a USB interface in the wrist band, it is greatly possible that the wrist band is covered by liquid due to being worn on the user's arm, and thus malfunction of the wrist band frequently occurs.

### SUMMARY

In order to solve the problem in the prior art that since it is required to provide a USB interface in the wrist band, it is greatly possible that the wrist band is covered by liquid due to being worn on the user's arm, and thus malfunction of the wrist band frequently occurs, the embodiments of the invention provides a wearable device and its manufacturing method, the technical solution is described as follows:

According to a first aspect of embodiments of the invention, there is provided a main body of a wearable device, comprising:
a front case, a bottom case and electronic components;
the front case is joined to the bottom case in an up-and-down direction, and the front case and the bottom case together enclose a space forming a waterproof cavity;
the electronic components are disposed in the cavity;
the electronic components comprise a printed circuit board (PCB) and a battery, which are electrically connected, and the PCB is provided with a wireless data transceiving assembly and at least one type of sensor;
two charging contacts are formed on the outer side of the bottom case and electrically connected with the electronic components.

In a particular embodiment, the main body further comprises a front case decorative piece;
the front case comprises an outer case surface which is partially or completely transparent;
the electronic components further comprise at least one indicator light electrically connected with the PCB, and the indicator light is disposed proximate to one side of the outer case surface within the cavity;
the front case decorative piece is attached to outer side of the outer case surface, and the front case decorative piece is provided with at least one light penetration area corresponding the indicator light.

In a particular embodiment, the front case decorative piece is a metal decorative piece, each light penetration area comprises several micro-apertures that extend through the upper and lower surfaces of the metal decorative piece and are arranged evenly, and light penetration fillers are provided in the micro-apertures.

In a particular embodiment, the front case decorative piece is formed with laser engraving hot melt glue lines on the surface facing the front case, and the front case decorative piece is glued to and connected with the outer surface of the front case via the hot melt glue.

In a particular embodiment, the front case is formed with a through hole for testing waterproofness, which is pasted with a waterproof sealing paste.

In a particular embodiment, after the front case is jointed with the bottom case, the outer case surface of the front case projects beyond jointing edges of the bottom case.

In a particular embodiment, the electronic components further comprise a vibration motor which is electrically connected to the PCB and the battery, respectively.

According to a second aspect of embodiments of the invention, there is provided a wearable device, comprising a main body and a flexible wrist band for accommodating the main body, and the main body is any main body according to the first aspect;
the flexible wrist band comprises a wrist band main body and a wrist buckle;
the wrist band main body has a center portion which is provided with an accommodating groove for accommodating the main body of the wearable device;
the wrist band main body has a first wrist band part and a second wrist band part respectively extending from either end of the accommodating groove;
the first wrist band part is provided with at least one fixed hole, and a terminal end of the first wrist band part is provided with an annular structure through which the second wrist band part is allowed to pass;
the second wrist band part is provided with a buckle hole;
the wrist band buckle embeds in the buckle hole, the wrist band buckle embeds in the fixed hole of the first wrist band part to secure the first wrist band part to the second wrist band part when a terminal end of the second wrist band part passes through the annular structure.

According to a third aspect of the invention, there is further provided a wearable device, comprising a main body and a charging cable for charging the main body, and the main body is any main body according to the first aspect;
the charging cable comprises a main body accommodating end and a charging end;
the main body accommodating end is formed with a semi-cavity for accommodating the main body of the wearable device, two charging terminals are formed on the inner surface of the semi-cavity and configured to electrically contact and connect with the two charging contacts of the main body when the main body of the wearable device is inserted into the semi-cavity;
the two charging terminals of the main body accommodating end are electrically connected with the charging end;
the charging end is at least one of a USB interface, a Mini USB interface, a Micro USB interface, a 30-pin interface and a power adapter.

According to a fourth aspect of the embodiments of the invention, there is provided a method for manufacturing a device, for manufacturing any main body of the wearable device according to the first aspect, comprising the steps of :
acquiring a front case and a bottom case of polycarbonate (PC) material by thermo-molding;
placing electronic components in a cavity enclosed by the front case and the bottom case, and electrically connecting the electronic components with two charging contacts on the bottom case;
waterproof sealing the cavity enclosed by the front case and the bottom case;
waterproof testing the cavity enclosed by the front case and the bottom case.

In a particular embodiment, the step of waterproof testing the cavity enclosed by the front case and the bottom case comprises the steps of:
forming a through hole on the front case;
waterproof testing the cavity by pressurized gas through the through hole;
sealing the through hole by using the waterproof sealing paste when the waterproof test succeeds.

In a particular embodiment, the method further comprises the steps of:
processing a metal sheet into a front case decorative piece;
attaching the front case decorative piece onto an outer case surface of the front case.

In a particular embodiment, the step of processing a metal sheet into a front case decorative piece, comprises the steps of:
processing the metal sheet into a rectangular sheet with rounded corners by press molding;
polishing, sandblasting and oxidating an outer surface of the rectangular sheet with rounded corners;
high-light polishing side edges of the rectangular sheet with rounded corners, and then oxidating the side edges for a second time after polishing;
milling light penetration areas of the rectangular sheet with rounded corners;
drilling a predetermined number of micro-apertures on each light penetration area;
injecting UV curing glue into the micro-apertures;
irradiating UV light to the micro-apertures, and curing the UV curing glue in the micro-apertures.

In a particular embodiment, the step of attaching the front case decorative piece onto an outer case surface of the front case, comprises the steps of:
laser engraving hot melt glue lines on a surface of the front case decorative piece facing the front case;
connecting the front case decorative piece to the outer case face of the front case via the hot melt glue.

The technical solution as provided by the embodiments of the invention may include the following advantageous effects:

By providing a waterproof cavity housing the wireless data transceiving assembly in the wearable device, it is solved the problem in the prior art that there is required to provide a USB interface, but it is greatly possible that the wrist band is covered by liquid due to being worn on the user's arm, and thus malfunction of the wrist band frequently occurs, thereby achieving such effect that the user may use and wear the wearable device in a humid environment or whenever needs to contact water.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate embodiments consistent with the invention and, together with the description, serve to explain the principles of the invention.
Fig. 1 is an exploded view showing a main body of a wearable device according to one exemplary embodiment;
Fig. 2 is an exploded view showing a main body of a wearable device according to another exemplary embodiment;
Fig. 3 is a schematic view showing a wearable device according to another exemplary embodiment;
Fig. 4 is a schematic view showing a wearable device according to another exemplary embodiment;
Fig. 5 is a flow chart of a method for manufacturing a main body of a wearable device according to another exemplary embodiment; and
Fig. 6 is a flow chart of a method for manufacturing a main body of a wearable device according to another exemplary embodiment.

Through the above accompanying drawings, the particular embodiments of the invention have been shown, for which a more detailed description will be given as below. These drawings and textual description are by no means intended to limit the scope of the concept of the invention, but to explain the concept of the invention to those skilled in the art through the particular embodiments.

### DESCRIPTION OF THE EMBODIMENTS

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the invention. Instead, they are merely examples of apparatuses and methods consistent with aspects related to the invention as recited in the appended claims.

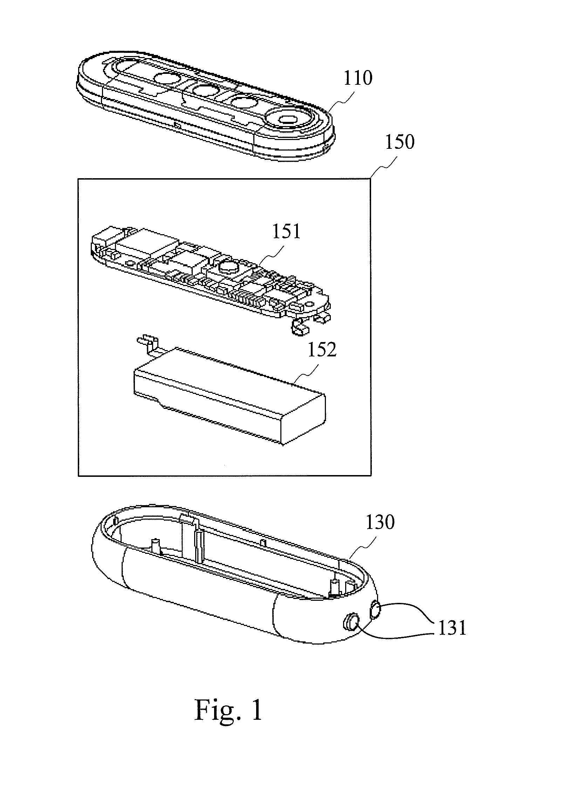

Fig. 1 an exploded view showing a main body of a wearable device according to an exemplary embodiment. The main body of the wearable device includes: a front case 110, a bottom case 130 and electronic components 150.

The front case 110 is joined to the bottom case 130 in an up-and-down direction, and the front case 110 and the bottom case 130 together enclose a space forming a waterproof cavity.

The electronic components 150 are disposed in the cavity.

The electronic components 150 include a PCB (Printed Circuit Board) 151 and a battery 152, which are electrically connected, and the PCB 151 is provided with a wireless data transceiving assembly and at least one type of sensor.

Two charging contacts 131 are formed on the outer side of the bottom case 130 and electrically connected with the electronic components 150.

Accordingly, the main body of the wearable device provided by this embodiment solves the problem in the prior art that it is required to provide a USB interface, but it is greatly possible that the wrist band is covered by liquid due to being worn on the user's arm, and thus malfunction of the wrist band frequently occurs, by providing a waterproof cavity housing the wireless data transceiving assembly in the wearable device, thereby achieving such effect that the user may use and wear the wearable device in a humid environment or whenever needs to contact water.

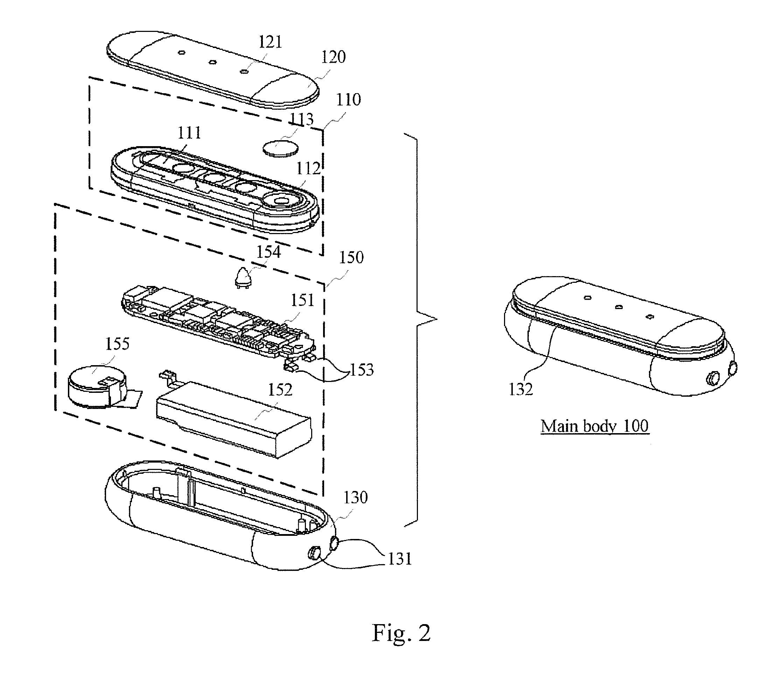

Fig. 2 is an exploded view showing a main body of a wearable device according to another exemplary embodiment. The main body of the wearable device includes: a front case 110, a bottom case 130 and electronic components 150.

The front case 110 is joined to the bottom case 130 in an up-and-down direction, and the front case 110 and the bottom case 130 together enclose a space forming a waterproof cavity.

The electronic components 150 are disposed in the cavity.

The electronic components 150 include a PCB 151 and a battery 152, which are electrically connected, and the PCB 151 is provided with a wireless data transceiving assembly and at least one type of sensor. The electronic components 150 are further provided with two mating contacts 153.

Two charging contacts 131 are formed on the outer side of the bottom case 130 and electrically connected with the electronic components 150, i.e., electrically connected with the mating contacts 153.

Alternatively, the main body further includes a front case decorative piece 120.

The front case 110 includes an outer case surface 111 which is partially or completely transparent.

The electronic components 150 further include at least one indicator light 154 electrically connected with the PCB 151, the indicator light 154 is disposed on one side of the outer case surface 111 within the cavity.

The front case decorative piece 120 is attached to the outer side of the outer case surface 111, and the front case decorative piece 120 is provided with at least one light penetration area 121 corresponding the indicator light 154.

The indicator light 154 disposed on one side proximate to the outer case surface 111 in the cavity directly face the light penetration area 121. That is, light radiated by the indicator light 154 is transmitted from the light penetration area 121 through the transparent portion of the outer case surface 111.

Alternatively, the front case decorative piece 120 is a metal decorative piece, each light penetration area 121 includes several micro-apertures that extend through the upper and lower surfaces of the metal decorative piece and are arranged evenly, and light penetration fillers are provided in the micro-apertures. The light penetration fillers may be UV (Ultraviolet Rays) glue. For example, the light penetration area is a round area with a diameter of 2.5mm, in which there are 170 micro-apertures each with a diameter of 0.07mm.

Alternatively, the front case decorative piece 120 is formed with laser engraving hot melt glue lines on the surface facing the front case 110, the front case decorative piece 120 is glued to and connected with the outer surface of the front case 110 via the hot melt glue. This embodiment does not limit the shape of the hot melt glue lines.

Alternatively, the front case 110 is formed with a through hole for testing waterproofness, which is pasted with a waterproof sealing paste 113.

Alternatively, after the front case 110 is jointed with the bottom case 130, the outer case surface 111 of the front case 110 projects beyond jointing edges 132 of the bottom case 130.

Alternatively, the electronic components 150 further include a vibration motor 155 which is electrically connected to the PCB 151 and the battery 152, respectively.

Accordingly, the main body of the wearable device provided by this embodiment solves the problem in the prior art that it is required to provide a USB interface, but it is greatly possible that the wrist band is covered by liquid due to being worn on the user's arm, and thus malfunction of the wrist band frequently occurs, by providing a waterproof cavity housing the wireless data transceiving assembly in the wearable device, thereby achieving such effect that the user may use and wear the wearable device in a humid environment or whenever needs to contact water.

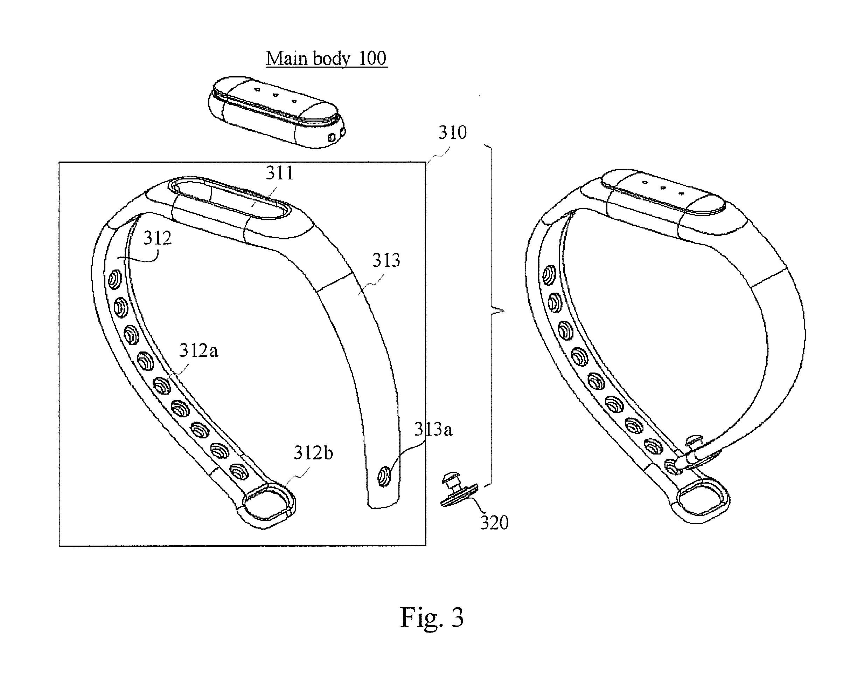

Fig. 3 is a schematic view showing a wearable device according to another exemplary embodiment. The wearable device includes a main body and a flexible wrist band for accommodating the main body, wherein,

The main body is any main body of the wearable device mentioned in the embodiments as shown in Fig. 1 or Fig. 2.

The flexible wrist band 300 includes a wrist band main body 310 and a wrist buckle 320.

The wrist band main body 310 has a center portion which is provided with an accommodating groove 311 for accommodating the main body of the wearable device.

The wrist band main body 310 respectively extends from both ends of the accommodating groove 311 to form a first wrist band part 312 and a second wrist band part 313.

The first wrist band part 312 is provided with at least one fixed hole 312a, and the terminal end of the first wrist band part 312 is provided with an annular structure 312b through which the second wrist band part 313 is allowed to pass.

The second wrist band part 313 is provided with a buckle hole 313a.

The wrist band buckle 320 embeds in the buckle hole 313a, the wrist band buckle 320 embeds in the fixed hole 312a of the first wrist band part 312 to secure the first wrist band part 312 to the second wrist band part 313 when a terminal end of the second wrist band part 313 passes through the annular structure 312b.

Accordingly, the main body of the wearable device provided by this embodiment solves the problem in the prior art that it is required to provide a USB interface, but it is greatly possible that the wrist band is covered by liquid due to being worn on the user's arm, and thus malfunction of the wrist band frequently occurs, by providing a waterproof cavity housing the wireless data transceiving assembly in the wearable device, thereby achieving such effect that the user may use and wear the wearable device in a humid environment or whenever needs to contact water.

It should be noted that the flexible wrist band for accommodating the main body provided by this embodiment prevents the charging contacts from being exposed, by receiving the main body in the accommodating groove, and the wearable device may be worn at a desired body portion via the wrist band main body.

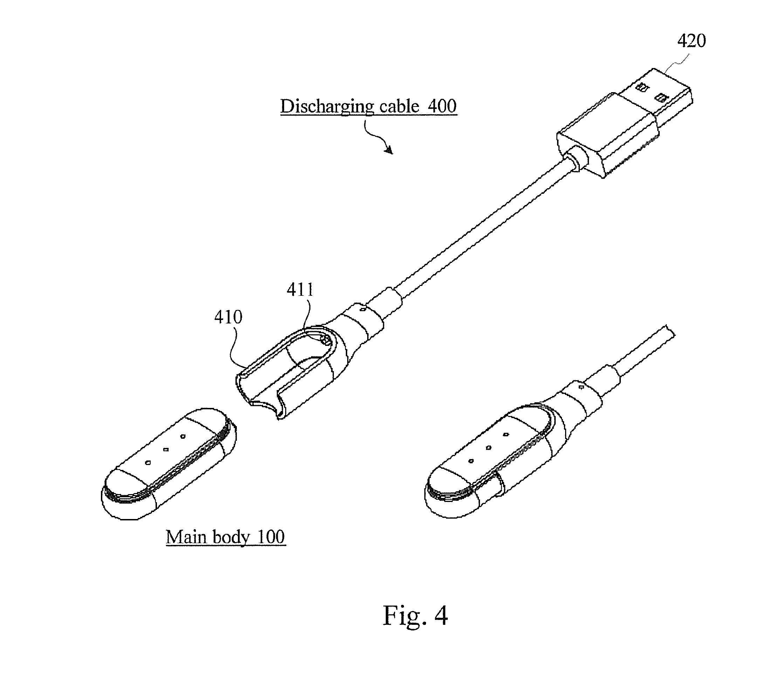

Fig. 4 is a schematic view showing a wearable device according to another exemplary embodiment. The wearable device includes a main body and a charging cable for charging the main body, wherein,

The main body may be any main body of the wearable device mentioned in the embodiments as shown in Fig. 1 or Fig. 2.

The charging cable 400 includes a main body accommodating end 410 and a charging end 420.

The main body accommodating end 410 is formed with a semi-cavity for accommodating the main body of the wearable device, two charging terminals 411 are formed on the inner surface of the semi-cavity and configured to electrically contact and connect with the two charging contacts 131 of the main body when the main body of the wearable device is inserted into the semi-cavity.

The two charging terminals 411 of the main body accommodating end 410 are electrically connected with the charging end 420.

The charging end 420 is at least one of a USB interface, a Mini USB (Mini Universal Serial Bus) interface, a Micro USB (Micro Universal Serial Bus) interface, a 30-pin interface and a power adapter. In this embodiment, the charging end 420 is a USB interface, for example.

Accordingly, the main body of the wearable device provided by this embodiment solves the problem in the prior art that it is required to provide a USB interface, but it is greatly possible that the wrist band is covered by liquid due to being worn on the user's arm, and thus malfunction of the wrist band frequently occurs, by providing a waterproof cavity housing the wireless data transceiving assembly in the wearable device, thereby achieving such effect that the user may use and wear the wearable device in a humid environment or whenever needs to contact water.

It should be noted that the charging cable for charging the main body provided by this embodiment is adapted to charge the main body of the wearable device, and through various ports and various kinds of charging ends.

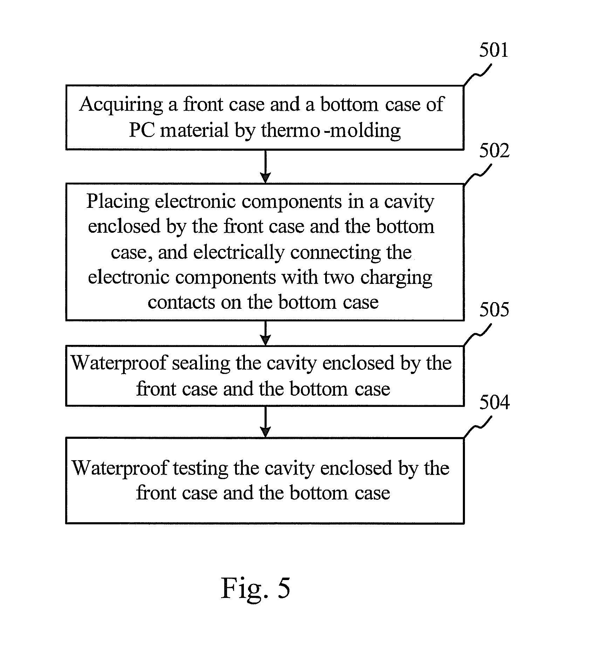

Fig. 5 is a flow chart of a method for manufacturing the main body of the wearable device according to one exemplary embodiment. The method for manufacturing any main body of the wearable device in the embodiment as shown in Fig. 1 or Fig. 2 includes:

In step 501, the front case and the bottom case of polycarbonate (PC) material are acquired by thermo-molding.

In step 502, the electronic components are placed in the cavity enclosed by the front case and the bottom case, and the electronic components are electrically connected with the two charging contacts on the bottom case.

In step 503, the cavity enclosed by the front case and the bottom case is sealed to be waterproof.

In step 504, the cavity enclosed by the front case and the bottom case is tested for waterproofness.

Accordingly, the method for manufacturing the wearable device provided by this embodiment solves the problem in the prior art that it is required to provide a USB interface, but it is greatly possible that the wrist band is covered by liquid due to being worn on the user's arm, and thus malfunction of the wrist band frequently occurs, by providing a waterproof cavity housing the wireless data transceiving assembly in the wearable device, thereby achieving such effect that the user may use and wear the wearable device in a humid environment or whenever needs to contact water.

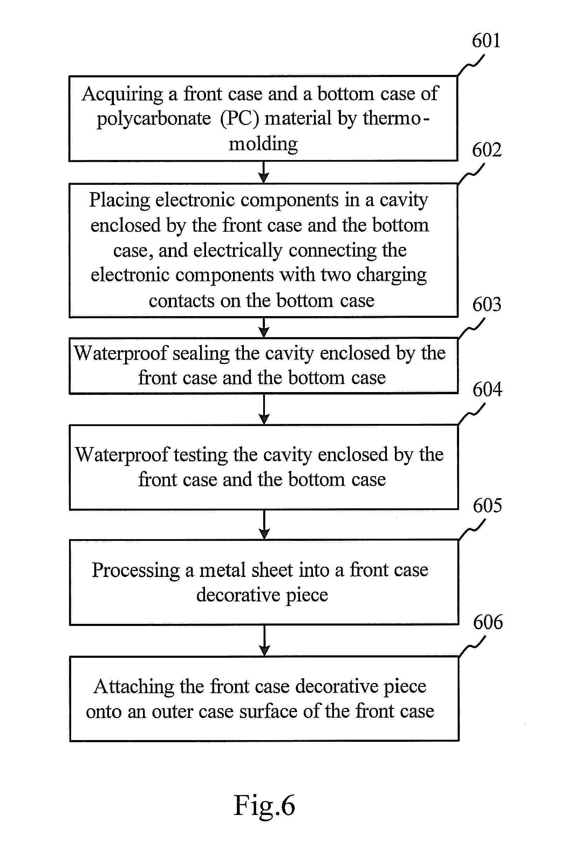

Fig. 6 is a flow chart of a method for manufacturing the main body of the wearable device according to one exemplary embodiment. The method for manufacturing any main body of the wearable device in the embodiment as shown in Fig. 1 or Fig. 2 includes:

In step 601, the front case and the bottom case of PC material are acquired by thermo-molding.

The front case includes an outer case surface in a shape of a rectangle with rounded corners and a case edge formed by extending along a direction perpendicular to the outer case surface from the edge thereof. The outer case surface is transparent, or may be partially or completely transparent.

In step 602, the electronic components are placed in the cavity enclosed by the front case and the bottom case, and the electronic components are electrically connected with the two charging contacts on the bottom case.

The electronic components are provided with two charging contacts which fit and contact with extending portions inside the two charging contacts on the outer side of the bottom case; and the electronic components are provided therein with PCB, a power motor, a battery, an indicator, wireless data transceiving assembly and at least one type of sensor.

In step 603, the cavity enclosed by the front case and the bottom case is sealed to be waterproof.

The cavity is sealed to be waterproof by using the sealing method, such as 0-shaped ring;

In step 604, the cavity enclosed by the front case and the bottom case is tested for waterproofness.

This step may be divided into three sub-steps:

firstly, a through hole is formed on the front case;

secondly, the cavity is tested for waterproofness by pressurized gas through the through hole, i.e., whether the test succeeds is determined depending on pressure loss;

thirdly, the through hole is sealed by using the waterproof sealing paste when the waterproof test succeeds.

In step 605, the metal sheet is processed into front case decorative piece.

This step may be divided into seven sub-steps:
firstly, the metal sheet is processed into a rectangular sheet with rounded corners by press molding, and the metal sheet may be aluminum alloy;
secondly, the outer surface of the rectangular sheet with rounded corners is polished, sandblasted and oxidated;
thirdly, side edges of the rectangular sheet with rounded corners is high-light polished, and then oxidated for a second time after polishing;
fourthly, the light penetration areas of the rectangular sheet with rounded corners is milled, and the lower portion of the light penetration area directly faces the indicator light positioned under the light penetration area of the outer case surface;
fifthly, a predetermined number of micro-apertures are drilled on each light penetration area, and laser is used to drill the micro-apertures;
sixthly, the UV curing glue is injected into the micro-apertures;
seventhly, UV light irradiates the micro-apertures to cure the UV curing glue in the micro-apertures.

In step 606, the front case decorative piece is attached onto the outer case surface of the front case.

This step may be divided into two sub-steps:
firstly, the hot melt glue lines is laser engraved on the surface of the front case decorative piece facing the front case;
secondly, the front case decorative piece is connected to the outer case face of the front case via the hot melt glue.

Accordingly, the method for manufacturing the wearable device provided by this embodiment solves the problem in the prior art that it is required to provide a USB interface, but it is greatly possible that the wrist band is covered by liquid due to being worn on the user's arm, and thus malfunction of the wrist band frequently occurs, by providing a waterproof cavity housing the wireless data transceiving assembly in the wearable device, thereby achieving such effect that the user may use and wear the wearable device in a humid environment or whenever needs to contact water.

It should be noted that light penetration of the light penetration area of the rectangular sheet with rounded corners is increased by milling the light penetration area of the rectangular sheet with rounded corners, so that the indicator light may operate with a lower brightness so as to reduce energy consumption of the indicator light.

It should be noted that bonding strength between the front case decorative piece and the front case is increased by laser engraving the hot melt glue lines on the surface of the front case decorative piece facing the front case.

Other embodiments of the invention will be apparent to those skilled in the art from consideration of the specification and practice of the invention disclosed here. This application is intended to cover any variations, uses, or adaptations of the invention following the general principles thereof and including such departures from the invention as come within known or customary practice in the art. It is intended that the specification and examples be considered as exemplary only, with a true scope and spirit of the invention being indicated by the following claims.

It will be appreciated that the present invention is not limited to the exact construction that has been described above and illustrated in the accompanying drawings.

## Claims

1. A main body of a wearable device, **characterized in that**, the main body comprises:
a front case (110), a bottom case (130) and electronic components (150);
wherein
the front case (110) is joined to the bottom case (130) in an up-and-down direction, and the front case (110) and the bottom case (130) together enclose a space forming a waterproof cavity;
the electronic components (150) are disposed in the cavity;
the electronic components (150) comprise a printed circuit board PCB (151) and a battery (152), the PCB and the battery (152) are electrically connected, and the PCB (151) is provided with a wireless data transceiving assembly and at least one type of sensor;
the bottom case (130) has two charging contacts (131) formed on the outer side thereof and electrically connected with the electronic components (150).

2. The main body according to claim 1, wherein, the main body further comprises a front case decorative piece (120);
the front case (110) comprises an outer case surface (111) which is partially or completely transparent;
the electronic components (150) further comprise at least one indicator light (154) electrically connected with the PCB (151), and the indicator light (154) is disposed proximate to one side of the outer case surface (111) within the cavity; and
the front case decorative piece (120) is attached to outer side of the outer case surface (111), and the front case decorative piece (120) is provided with at least one light penetration area (121) corresponding the indicator light (154).

3. The main body according to claim 2, wherein, the front case decorative piece (120) is a metal decorative piece, each light penetration area (121) comprises several micro-apertures that extend through the upper and lower surfaces of the metal decorative piece and are arranged evenly, and light penetration fillers are provided in the micro-apertures.

4. The main body according to claim 2 or 3, wherein, the front case decorative piece (120) is formed with laser engraving hot melt glue lines on the surface facing the front case (110), and the front case decorative piece (120) is glued to and connected with the outer surface of the front case (110) via the hot melt glue.

5. The main body according to any one of claims 1-4, wherein, the front case (110) is formed with a through hole for testing waterproofness, which is pasted with a waterproof sealing paste.

6. The main body according to any one of claims 1-5, wherein, after the front case (110) is jointed with the bottom case (130), the outer case surface (111) of the front case (110) projects beyond jointing edges of the bottom case (130).

7. The main body according to any one of claims 1-6, wherein, the electronic components (150) further comprise a vibration motor which is electrically connected to the PCB (151) and the battery (152), respectively.

8. A wearable device, **characterized in that**, the wearable device comprises a main body and a flexible wrist band (300) for accommodating the main body (310);
wherein
the main body is the main body according to any one of claims 1-7;
the flexible wrist band (300) comprises a wrist band main body (310) and a wrist buckle (320);
the wrist band main body (310) has a center portion which is provided with an accommodating groove (311) for accommodating the main body of the wearable device;
the wrist band main body (310) has a first wrist band part (312) and a second wrist band part (313) respectively extending from either end of the accommodating groove (311);
the first wrist band part (312) is provided with at least one fixed hole (312a), and a terminal end of the first wrist band part (312) is provided with an annular structure (312b) through which the second wrist band part (313) is allowed to pass;
the second wrist band part (313) is provided with a buckle hole (313a);
the wrist band buckle (320) embeds in the buckle hole (313a), the wrist band buckle (320) embeds in the fixed hole (312a) of the first wrist band part (312) to secure the first wrist band part (312) to the second wrist band part (313) when a terminal end of the second wrist band part (313) passes through the annular structure (312b).

9. A wearable device, **characterized in that**, the wearable device comprises a main body and a charging cable (400) for charging the main body;
wherein
the main body is the main body according to any one of claims 1-7;
the charging cable (400) comprises a main body accommodating end (410) and a charging end (420);
the main body accommodating end (410) is formed with a semi-cavity for receiving the main body of the wearable device, two charging terminals (411) are formed on the inner surface of the semi-cavity and configured to electrically contact and connect with the two charging contacts (131) of the main body when the main body of the wearable device is inserted into the semi-cavity;
the two charging terminals (411) of the main body accommodating end (410) are electrically connected with the charging end (420);
the charging end (420) is at least one of a Universal Serial Bus USB interface, a Mini USB interface, a Micro USB interface, a 30-pin interface and a power adapter.

10. A method for manufacturing a device, for manufacturing the main body of the wearable device according to any one of claims 1-7, **characterized in that**, the method comprises the steps of:
acquiring (501) a front case and a bottom case of polycarbonate PC material by thermo-molding;
placing (502) electronic components in a cavity enclosed by the front case and the bottom case, and electrically connecting the electronic components with two charging contacts on the bottom case;
waterproof sealing (503) the cavity enclosed by the front case and the bottom case;
waterproof testing (504) the cavity enclosed by the front case and the bottom case.

11. The method according to claim 10, wherein, the step of waterproof testing (504) the cavity enclosed by the front case and the bottom case comprises the steps of:
forming a through hole on the front case;
waterproof testing the cavity by pressurized gas through the through hole;
sealing the through hole by waterproof sealing paste when the waterproof test succeeds.

12. The method according to claim 10 or 11, wherein, the method further comprises the steps of:
processing a metal sheet into a front case decorative piece;
attaching the front case decorative piece onto an outer case surface of the front case.

13. The method according to claim 12, wherein, the step of processing a metal sheet into a front case decorative piece comprises the steps of:
processing the metal sheet into a rectangular sheet with rounded corners by press molding;
polishing, sandblasting and oxidating an outer surface of the rectangular sheet with rounded corners;
high-light polishing side edges of the rectangular sheet with rounded corners, and then oxidating the side edges for a second time after polishing;
milling light penetration areas of the rectangular sheet with rounded corners;
drilling a predetermined number of micro-apertures on each light penetration area;
injecting ultraviolet UV curing glue into the micro-apertures;
irradiating UV light to the micro-apertures, and curing the UV curing glue in the micro-apertures.

14. The method according to claim 12 or 13, wherein, the step of attaching the front case decorative piece onto an outer case surface of the front case comprises the steps of:
laser engraving hot melt glue lines on a surface of the front case decorative piece facing the front case;
connecting the front case decorative piece to the outer case face of the front case via the hot melt glue.
